# EUROPEAN PATENT SPECIFICATION

(11) **EP 3 164 077 B1**
(45) Date of publication and mention of the grant of the patent: **11.09.2019**
(21) Application number: 15750457.2
(22) Date of filing: 02.07.2015
(51) Int. Cl.: A61B 8/00

(54) **PORTABLE ULTRASOUND INTERFACE FOR ULTRASOUND WORKSTATIONS**
TRAGBARE ULTRASCHALL-SCHNITTSTELLE FÜR ULTRASCHALL-ARBEITSSTATIONEN
INTERFACE À ULTRASONS PORTABLE POUR STATIONS DE TRAVAIL À ULTRASONS

(30) Priority: 03.07.2014 US 201462020572 P
(43) Date of publication of application: 10.05.2017
(73) Proprietor: Koninklijke Philips N.V., 5656 AE Eindhoven (NL)
(72) Inventor: TAHMASEBI MARAGHOOSH, Amir Mohammad, 5656 AE Eindhoven (NL)
(74) Representative: Philips Intellectual Property & Standards
(86) International application number: PCT/IB2015/054978
(87) International publication number: WO 2016/001865

(56) References cited:
- WO-A1-2008/068710
- WO-A1-2014/041503
- WO-A2-2006/111873
- WO-A2-2007/130526
- CN-A- 102 930 170
- JP-A- 2009 153 917
- JP-A- 2014 117 360

## Description

The present invention generally relates to an ultrasound workstation serving as a primary source for generating and/or displaying ultrasound images via an ultrasound transducer. The present invention specifically relates to an ultrasound tablet interfacing with the ultrasound workstation to serve as an alternative primary source or a secondary source for generating and/or displaying the ultrasound images.

In a typical sonography session, a patient is lying or sitting on a bed, and an ultrasound workstation is set besides the patient. The sonographer holds an ultrasound transducer next to the patient's body while setting a number of imaging parameters as desired to generate and display ultrasound images of a particular anatomical region of the patient. More particularly, the sonographer will have one hand holding the ultrasound transducer on the patient's body and the other hand adjusting imaging parameters on the ultrasound workstation. However, while the eye-hand coordination of the sonographer in generating optimal ultrasound images of the anatomical region of interest is inconveniently limited by a degree of which a field of view of the sonographer is exclusive of either the ultrasound transducer or a display of the ultrasound workstation.

JP 2009/153917, WO 2014/041503 and JP 2014/117360 each disclose an ultrasonic diagnostic apparatus with a portable display device.

The invention is defined by the claims.

The present invention provides an ultrasound tablet interfacing with an ultrasound workstation to facilitate a field of view of the sonographer being inclusive of both the ultrasound transducer and a display of the ultrasound tablet. For purposes of the present invention, (1) the term "ultrasound transducer" is broadly defined herein as any device structurally configured for transmitting and receiving ultrasound waves to and from an anatomical region of a patient, (2) the term "ultrasound workstation" is broadly defined herein as any computer structurally configured to interface with an ultrasound transducer for purposes of generating and/or displaying ultrasound images of the anatomical region, and (3) the term "ultrasound tablet" is broadly defined herein as any portable computer structurally configured in a single panel with a touchscreen as an input device to interface with an ultrasound workstation for purposes of generating and/or displaying ultrasound images of the anatomical region.

One form of the present invention is an ultrasound system employing an ultrasound transducer, an ultrasound workstation and an ultrasound tablet. In operation, the ultrasound transducer generates ultrasound signals representative of an ultrasound imaging of an anatomical region, the ultrasound workstation generates an ultrasound image of the anatomical region in accordance with a user interface with the ultrasound workstation and/or the ultrasound tablet, and the ultrasound tablet displays a portion or an entirety of the ultrasound image of the anatomical region in accordance with a user interface with the ultrasound workstation and/or the ultrasound tablet. The ultrasound tablet is attachable to and detachable from the ultrasound workstation.

The foregoing form and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims and equivalents thereof.
FIG. 1 illustrates an exemplary embodiment of an ultrasound system in accordance with the present invention.
FIG. 2 illustrates exemplary embodiments of an ultrasound workstation and an ultrasound tablet in accordance with the present invention.

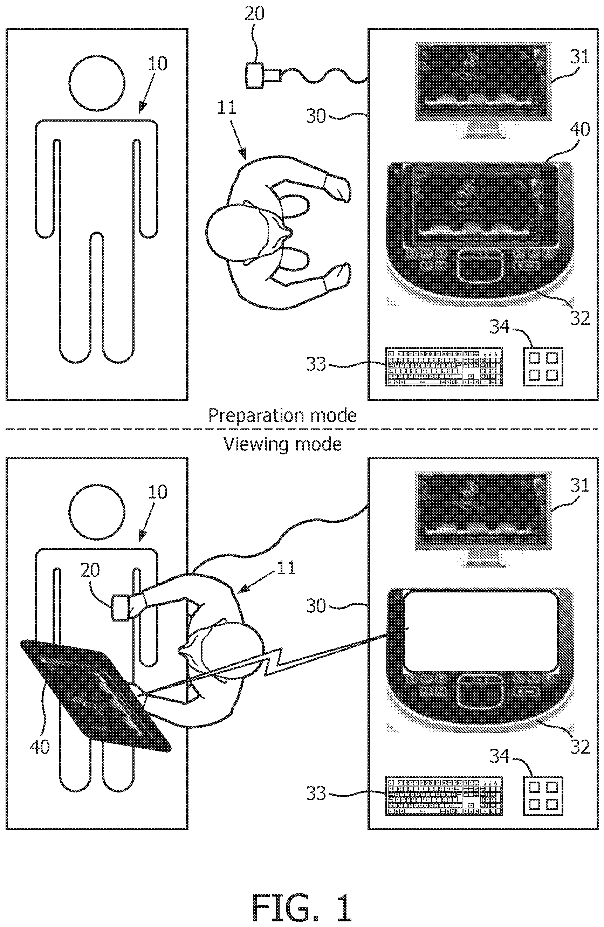

Referring to FIG. 1, a sonography session of a patient 10 executed by a sonographer 11 utilizes an ultrasound system of the present invention employing an ultrasound transducer 20, an ultrasound workstation 30 and an ultrasound tablet 40. In practice, ultrasound transducer 20 may be operated by sonographer 11 to generate a planar image or a volumetric image of an anatomical region of patient 10, ultrasound workstation 30 may be mobile or stationary relative to patient 10, and ultrasound tablet 40 is selectively attachable and detachable from a hard key platform 32 of ultrasound workstation 30.

As shown, the sonography session has a preparation mode and a viewing mode. For purposes of generally describing the preparation mode and the viewing mode, only user interface components of ultrasound workstation 30 including hard key platform 32, a keyboard 33 and a soft key touchscreen panel 34 are shown. Please note components 31-34 and ultrasound tablet 40 are not drawn to scale, but sized to emphasize hard key platform 32 and ultrasound tablet 40.

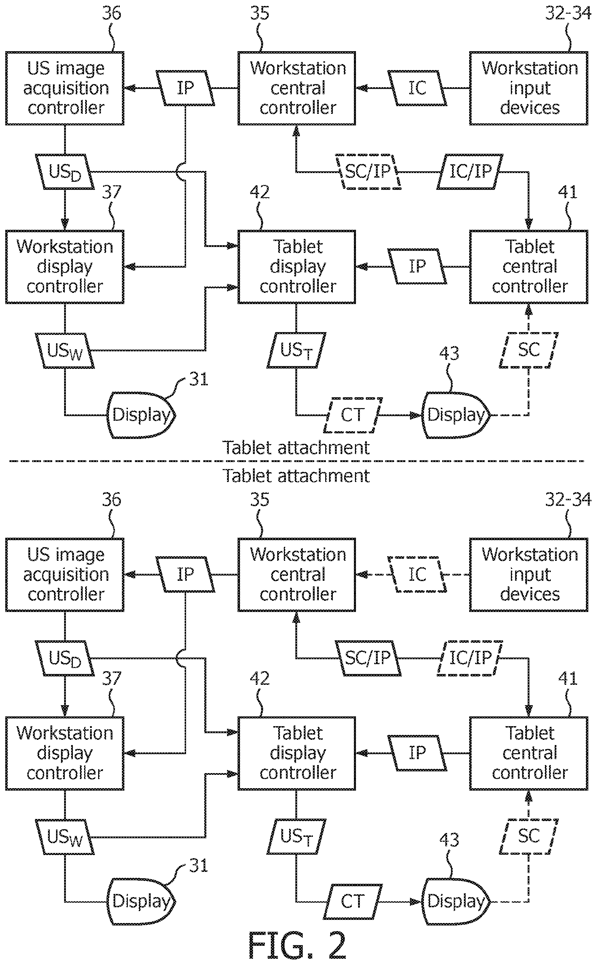

The preparation mode of the sonography session is based on ultrasound tablet 40 being attached to hard key platform 32 whereby ultrasound workstation 30 executes a wired communication of ultrasound data/images to ultrasound tablet 40 for the alternative primary or secondary display of ultrasound images by ultrasound tablet 40. The preparation mode generally involves sonographer 11 interfacing with components 32-34 to simultaneously or individually adjust imaging parameters of display 31 and the display of ultrasound tablet 40 as attached to hard key platform 32. Further alternatively or optionally, sonographer 11 may interface with a control touchscreen (not shown) (e.g., keyboard and/or key pad) of ultrasound tablet 40 to simultaneously or individually adjust imaging parameters of display 31 and the display of ultrasound tablet 40 as will be further described herein in connection with FIG. 2.

The viewing mode of the sonography session is based on ultrasound tablet 40 being detached from hard key platform 32 whereby ultrasound workstation 30 executes a wireless communication of ultrasound data/images to ultrasound tablet 40 for the alternative primary or secondary display of ultrasound images by ultrasound tablet 40. The viewing mode of the sonography session generally involves sonographer 11 using one hand to hold ultrasound transducer 20 on the body of patient 10, and the other hand to hold ultrasound tablet 40 to view a display of ultrasound images within and/or adjacent to a field of view of ultrasound transducer 20. Additionally, sonographer 11 may adjust imaging parameters on ultrasound tablet 40 as further described herein in connection with FIG. 2. Furthermore, sonographer 11 may utilize known features of a tablet including, but not limited to, a capability of zooming-in and/or zooming-out the ultrasound image via finger pointing on the control touchscreen of ultrasound tablet 40.

In practice, the preparation mode and the viewing mode of the sonography session as generally described herein may be specifically executed in numerous variations as would be appreciated by those having ordinary skill in the art. To facilitate further understanding of an interfacing ultrasound workstation 30 and ultrasound tablet 40 during a sonography session, FIG. 2 illustrates operation of controllers 35-37 of ultrasound workstation 30 (FIG. 1) and controllers 41 and 42 of ultrasound tablet 40 with ultrasound tablet 40 (FIG. 2) being attached and detached from ultrasound workstation 30.

Referring to FIG. 2, a workstation central controller 35 of ultrasound workstation 30 is structurally configured to control all operations of ultrasound workstation 30 including, but not limited to, a processing of input commands IC from a user interface of hard keys and/or soft keys of workstation input devices 32-34 (FIG. 1) to adjust image parameters IP as known in the art. Examples of image parameters IP include, but are not limited to, a depth, a resolution, a focus, a gain, a greyscale mode, a flow mode and a color power angio.

An ultrasound image acquisition controller 36 of ultrasound workstation 30 is structurally configured to generate ultrasound data US_{D} from a processing of ultrasound signals received from ultrasound transducer 20 (FIG. 1) including, but not limited to, beamforming, filtering, information detection and scan conversion of the ultrasound signals in accordance with image parameters IP received from workstation central controller 35.

A workstation display controller 37 of ultrasound workstation 30 is structurally configured to generate an ultrasound image USw for ultrasound display 31 from a processing of ultrasound data US_{D} including, but not limited to, a storage of ultrasound data US_{D}, a generation of necessary drive signals for ultrasound display 31 and an overlay of graphical information on ultrasound image USw (e.g., system configuration/operating information, a patient identification, a time/date stamp, etc.)

A tablet central controller 41 of ultrasound tablet 40 is structurally configured to control all operations of ultrasound tablet 40 including, but not limited to, a processing of soft key commands SC from a user interface of soft keys of touchscreen display 43 to adjust image parameters IP as known in the art.

A tablet display controller 42 of ultrasound tablet 40 is structurally configured to generate an ultrasound image US_{T} for ultrasound display 43 from a processing of ultrasound data US_{D} and/or ultrasound image USw including, but not limited to, a storage of ultrasound data US_{D} and/or ultrasound image USw, a generation of necessary drive signals for ultrasound display 43, an overlay of graphical information on the ultrasound image US_{T}, and a generation of a control panel screen CP of soft keys for user interface.

Central controllers 35 and 41 are further structurally configured to establish a wired communication responsive to ultrasound tablet 40 being attached to ultrasound workstation 30, and to establish a wireless communication responsive to ultrasound tablet 40 being detached from ultrasound workstation 30. These communications facilitate a transfer/exchange of data and signals as needed between ultrasound workstation 30 and ultrasound tablet 40.

Of particular importance to the present invention is (1) a transfer of ultrasound data US_{D} and/or ultrasound image US_{W} from respective controllers 36 and/or 37 to controller 42 as controlled by central controllers 35 and 41, and (2) an exchange of input commands IC/soft key commands SC/input parameters IP between central controllers 35 and 41. More particularly as to the command/parameter exchange, central controller 35 and/or central controller 41 contain a mapping of input commands IC and soft key commands SC as known in the art to facilitate a command interpretation by the central controller(s).

In practice, controllers 35-37 may be implemented by hardware, software, firmware and/or circuitry of ultrasound workstation 30 as will be appreciated by those having ordinary skill in the art, and controllers 41 and 42 may be implemented by hardware, software, firmware and/or circuitry of ultrasound tablet 40 as will be appreciated by those having ordinary skill in the art.

Still referring to FIG. 2, an attachment of ultrasound tablet 40 to ultrasound workstation 30 generally involves:
(1) Ultrasound acquisition controller 36 providing ultrasound data US_{D} to workstation display controller 37 in accordance with image parameters IP from workstation central controller 35 as directed by input commands IC from workstation input devices 32-34 to facilitate a desired display 31 of ultrasound image US_{W};
(2) A wired (or wireless) communication of input commands IC/image parameters IP from workstation central controller 35 to tablet central controller 41 and a wired (or wireless) communication of ultrasound data US_{D} /ultrasound image US_{W} from US image acquisition controller 36 and/or workstation display controller 37 to tablet display controller 42 to facilitate a desired display 43 of ultrasound image US_{T} in accordance with image parameters IP as directed by input commands IC from workstation input devices 32-34 (ultrasound image US_{T} being a highlighted portion or an entirety of ultrasound image US_{W)};
(3) Alternatively or optionally, a desired display 43 of control touchscreen CT providing a user interface of soft keys to generate soft key commands SC to adjust image parameters IP for tablet display controller 42; and
(4) Alternatively or optionally, a wired (or wireless) communication of generated soft key commands SC/image parameters IP from tablet central controller 41 to workstation central controller 35 to adjust image parameters IP for US image acquisition controller 36 and/or workstation display controller 37.

A subsequent detachment of ultrasound tablet 40 from ultrasound workstation 30 generally involves:
(1) A wireless communication of ultrasound data US_{D} /ultrasound image US_{W} from US image acquisition controller 36 and/or workstation display controller 37 to tablet display controller 42 to facilitate a desired display 43 of ultrasound image US_{T} in accordance with image parameters IP as directed by soft key commands SC (ultrasound image US_{T} being a highlighted portion or an entirety of ultrasound image US_{W)};
(2) A wireless communication of soft key commands SC/image parameters IP from tablet central controller 41 to workstation central workstation 35 to facilitate a desired display 31 of ultrasound image US_{W} in accordance with image parameters IP as directed by soft key commands SC; and
(3) Alternatively or optionally, a wireless communication of soft key commands SC/image parameters IP from workstation central controller 35 to tablet central controller 41 to facilitate a desired display 43 of ultrasound image US_{T} in accordance with image parameters IP as directed by input commands IC (ultrasound image US_{T} being a highlighted portion or an entirety of ultrasound image US_{W)}.

In practice, the operation of ultrasound workstation 30 and ultrasound tablet 40 as generally described in connection with FIG. 2 may be specifically executed in numerous variations as would be appreciated by those having ordinary skill in the art. For example, workstation input devices 32-34 may be exclusively operated to control image parameters IP for US image acquisition controller 36 while control touchscreen CT may be exclusively utilized to control image parameters IP for display controllers 37 and 42.

Referring to FIGS. 1 and 2, those having ordinary skill in the art will appreciate numerous benefits of an ultrasound tablet of the present invention including, but not limited to, (1) remote access to functional buttons of an ultrasound workstation; (2) remote visualization of a display of an ultrasound workstation to thereby facilitate a closer look for both a sonographer and a patient; (3) a capability to change image parameters via a touch-screen while directly looking at the image and the outcome; (4) a capability to perform direct measurements on the ultrasound image (e.g., size of tumor, circumference of anatomy) rather than by conventional approach which is by mouse clicks with a good hand-eye coordination; (5) a touch-screen-based volume rendering of ultrasound data with a capability to zoom-in/zoom-out with finger pointing; (6) a quick share of ultrasound image data with another clinician in the same or another room for a second opinion, and (7) the display of an ultrasound tablet having extra screens for additional features.

While various embodiments of the present invention have been illustrated and described, it will be understood by those skilled in the art that the embodiments of the present invention as described herein are illustrative, and various changes and modifications may be made and equivalents may be substituted for elements thereof without departing from the true scope of the present invention. In addition, many modifications may be made to adapt the teachings of the present invention without departing from its central scope. Therefore, it is intended that the present invention not be limited to the particular embodiments disclosed as the best mode contemplated for carrying out the present invention, but that the present invention includes all embodiments falling within the scope of the appended claims.

## Claims

1. An ultrasound system, comprising:
an ultrasound transducer (20) operable to generate ultrasound signals representative of an ultrasound imaging of an anatomical region;
an ultrasound workstation (30) comprising:
input devices forming a user interface for receiving input commands;
an ultrasound image acquisition controller (36);
a display (31); and
a display controller (37),
wherein the workstation (30) is operable in communication with the ultrasound transducer (20) to generate an ultrasound image of the anatomical region in accordance with image parameters for the ultrasound image acquisition controller (36) as directed by input commands provided to the user interface; and
an ultrasound tablet (40) comprising:
a control touchscreen (43); and
a display controller (42),
wherein the tablet (40) is operable in communication with the ultrasound workstation (30) to display at least a portion of the ultrasound image of the anatomical region as generated by the ultrasound workstation (30) in accordance with image parameters for the display controllers (37,42) as directed by input commands provided to the control touchscreen,
wherein the ultrasound tablet (40) is attachable to and detachable from the ultrasound workstation (30) and wherein the control touchscreen is operable exclusively to control image parameters for the display controllers (37,42).

2. The ultrasound system of claim 1, wherein the ultrasound tablet (40) is in wireless communication with the ultrasound workstation (30) responsive to being detached from the ultrasound workstation (40).

3. The ultrasound system of claim 1, wherein the ultrasound tablet (40) is in wired communication with the ultrasound workstation (30) responsive to being attached to the ultrasound workstation (40).

4. The ultrasound system of claim 1, wherein the ultrasound workstation (30) is further operable in communication with the ultrasound tablet (40) to display the ultrasound image of the anatomical region in accordance with the user interface with the ultrasound tablet (40).

5. The ultrasound system of claim 1, wherein the ultrasound tablet (30) is further operable in communication with the ultrasound workstation (30) to display the at least a portion of the ultrasound image of the anatomical region in accordance with the user interface with the ultrasound workstation (30).

## Patentansprüche

1. Ultraschallsystem, umfassend:
einen Ultraschallwandler (20), der betriebsbereit ist, um Ultraschallsignale zu erzeugen, die für eine Ultraschallbildgebung einer anatomischen Region repräsentativ sind;
eine Ultraschall-Workstation (30), umfassend:
Eingabevorrichtungen, die eine Benutzeroberfläche zum Empfangen von Eingabebefehlen bilden;
eine Ultraschallbilderfassungssteuerung (36);
eine Anzeige (31); und
eine Anzeigensteuerung (37),
wobei die Workstation (30) in Verbindung mit dem Ultraschallwandler (20) betriebsbereit ist, um ein Ultraschallbild der anatomischen Region in Übereinstimmung mit Bildparametern für die Ultraschallbilderfassungssteuerung (36) zu erzeugen, wie durch an die Benutzeroberfläche bereitgestellte Eingabebefehle angewiesen; und
ein Ultraschall-Tablet (40), umfassend:
einen Steuerungstouchscreen (43); und
eine Anzeigensteuerung (42),
wobei das Tablet (40) in Verbindung mit der Ultraschall-Workstation (30) betriebsbereit ist, um mindestens einen Teil des Ultraschallbildes der anatomischen Region anzuzeigen, wie es durch die Ultraschall-Workstation (30) in Übereinstimmung mit Bildparametern für die Anzeigensteuerungen (37, 42), wie durch an die Benutzeroberfläche bereitgestellte Eingabebefehle angewiesen, erzeugt wird,
wobei das Ultraschall-Tablet (40) an der Ultraschall-Workstation (30) befestigbar und von ihr ablösbar ist und wobei der Steuerungstouchscreen ausschließlich betriebsbereit ist, um Bildparameter für die Anzeigensteuerungen (37, 42) zu steuern.

2. Ultraschallsystem nach Anspruch 1, wobei das Ultraschall-Tablet (40) in drahtloser Verbindung mit der Ultraschall-Workstation (30) steht, wenn es von der Ultraschall-Workstation (30) abgelöst ist.

3. Ultraschallsystem nach Anspruch 1, wobei das Ultraschall-Tablet (40) in drahtgebundener Verbindung mit der Ultraschall-Workstation (30) steht, wenn es an der Ultraschall-Workstation (30) befestigt ist.

4. Ultraschallsystem nach Anspruch 1, wobei die Ultraschall-Workstation (30) weiter in Verbindung mit dem Ultraschall-Tablet (40) betriebsbereit ist, um das Ultraschallbild der anatomischen Region in Übereinstimmung mit der Benutzeroberfläche mit dem Ultraschall-Tablet (40) anzuzeigen.

5. Ultraschallsystem nach Anspruch 1, wobei das Ultraschall-Tablet (40) weiter in Verbindung mit der Ultraschall-Workstation (30) betriebsbereit ist, um den mindestens einen Teil des Ultraschallbildes der anatomischen Region in Übereinstimmung mit der Benutzeroberfläche mit der Ultraschall-Workstation (30) anzuzeigen.

## Revendications

1. Système à ultrasons, comprenant :
un transducteur à ultrasons (20) servant à générer des signaux ultrasonores représentatifs d'une imagerie à ultrasons d'une région anatomique ;
une station de travail à ultrasons (30) comprenant :
des dispositifs d'entrée formant une interface utilisateur pour recevoir des commandes d'entrée ;
un dispositif de commande d'acquisition d'images ultrasonores (36) ;
un dispositif d'affichage (31) ; et
un dispositif de commande de dispositif d'affichage (37),
dans lequel la station de travail (30) peut fonctionner en communication avec le transducteur à ultrasons (20) pour générer une image ultrasonore de la région anatomique conformément à des paramètres d'image pour le dispositif de commande d'acquisition d'images ultrasonores (36) tels qu'indiqués par les commandes d'entrée fournies à l'interface utilisateur ; et
une tablette à ultrasons (40) comprenant :
un écran tactile de commande (43) ; et
un dispositif de commande de dispositif d'affichage (42),
dans lequel la tablette (40) peut fonctionner en communication avec la station de travail à ultrasons (30) pour afficher au moins une partie de l'image ultrasonore de la région anatomique telle que générée par la station de travail à ultrasons (30) conformément à des paramètres d'image pour les dispositifs de commande de dispositif d'affichage (37, 42) tels qu'indiqués par les commandes d'entrée fournies à l'écran tactile de commande,
dans lequel la tablette à ultrasons (40) peut être fixée à la station de travail à ultrasons (30) et détachée de celle-ci et dans lequel l'écran tactile de commande peut fonctionner exclusivement pour commander les paramètres d'image pour les dispositifs de commande de dispositif d'affichage (37, 42).

2. Système à ultrasons selon la revendication 1, dans lequel la tablette à ultrasons (40) est en communication sans fil avec la station de travail à ultrasons (30) en réponse au fait d'être détachée de la station de travail à ultrasons (30).

3. Système à ultrasons selon la revendication 1, dans lequel la tablette à ultrasons (40) est en communication filaire avec la station de travail à ultrasons (30) en réponse au fait d'être fixée à la station de travail à ultrasons (30).

4. Système à ultrasons selon la revendication 1, dans lequel la station de travail à ultrasons (30) peut en outre fonctionner en communication avec la tablette à ultrasons (40) pour afficher l'image ultrasonore de la région anatomique conformément à l'interface utilisateur avec la tablette à ultrasons (40).

5. Système à ultrasons selon la revendication 1, dans lequel la tablette à ultrasons (40) peut en outre fonctionner en communication avec la station de travail à ultrasons (30) pour afficher l'au moins une partie de l'image ultrasonore de la région anatomique conformément à l'interface utilisateur avec la station de travail à ultrasons (30).
